# EUROPEAN PATENT SPECIFICATION

(11) **EP 0 705 146 B1**
(45) Date of publication and mention of the grant of the patent: **11.03.1998**
(21) Application number: 94918477.4
(22) Date of filing: 23.06.1994
(51) Int. Cl.: B05B 17/06, A61M 15/00

(54) **ATOMISING DISPENSER**
ZERSTÄUBER
DIFFUSEUR-PULVERISATEUR

(30) Priority: 23.06.1993 GB 9312984
(43) Date of publication of application: 10.04.1996
(73) Proprietor: BESPAK PLC, King's Lynn Norfolk PE30 2JJ (GB)
(72) Inventor: FRY, Andrew Robert, 13 Greenhill Park, Hertfordshire EN5 1HQ (GB); MARSH, John David, 71 North End, Royston Hertfordshire SG8 5NZ (GB); BARNES, Paul, King's Lynn Norfolk PE32 1RL (GB)
(74) Representative: Bucks, Teresa Anne
(86) International application number: PCT/GB94/01357
(87) International publication number: WO 95/00254

(56) References cited:
- DE-A- 2 450 801
- GB-A- 922 310
- US-A- 3 473 530
- US-A- 5 113 855
- DATABASE WPI 24 March 1982 Derwent Publications Ltd., London, GB; AN B4027 & SU,A,825 176 (ODESS. UNIV.) 10 May 1981

## Description

This invention relates to a method of dispensing a flowable material in which a quantity of the material is entrained in a flow of gas and is atomised.

A particular use for such atomisation is in the dispensing of medicinal products where liquid or particulate material should preferably be finely atomised prior to inhalation, it being a known characteristic of inhalation therapy that its effectiveness is critically dependent upon the material being finely atomised in a controlled manner.

Other applications in which atomisation is important include the atomisation of fuel in fuel burners and internal combustion engines and the atomisation of water in humidifiers.

USSR Patent specification 825176 describes a device for spraying or atomising liquids or other fluids using ultrasound oscillations. Compressed gas is directed through a central tube onto a membrane which this causes to distort and oscillate. The fluid to be atomised is fed down a coaxial tube around the outside of the gas tube to the surface of the diaphragm in the form of a film. The gas disperses the film peripherally which is atomised on contact with the diaphragm.

It is an object of the present invention to provide an improved method and apparatus for atomising liquids and particulate solids which provides atomisation in a controlled repeatable fashion together with entrainment of the atomised material in a flow of gas for delivery to a required site.

According to the present invention there is disclosed a method of dispensing a flowable material comprising the steps of supplying pressurised gas to a nozzle such that a flow of gas is discharged through an aperture defined between a lip of the nozzle and a diaphragm, introducing the material into the flow of gas such that material is entrained in the flow, and vibrating the diaphragm so as to atomise the material, the diaphragm constituting a wall of a chamber communicating with the aperture, the diaphragm being deformable in response to excess gas pressure in the chamber from a rest position to a displaced position in which the aperture is opened to receive the flow passing between the lip and the diaphragm, the pressurised gas being supplied via the chamber such that the diaphragm is deformed into the displaced position and the diaphragm being peripherally clamped such that it is maintained under tension in the displaced position whereby the diaphragm is vibrated by the flow of gas, characterised in that the diaphragm has an outer portion which defines said wall of the chamber, the lip of the nozzle being annular thereby defining a mouth of an outlet defined by the nozzle and which is traversed by a central portion of the diaphragm in the rest position, and wherein the method includes the step of biasing the diaphragm into contact with the lip when the diaphragm is in its rest position.

It has been observed that the effect of the vibrating diaphragm on the dispensed material is that atomisation is enhanced. In the case of liquid droplets, a fine mist can be produced in a repeatable fashion and by adjusting the dynamic characteristics of the diaphragm the distribution of droplet size can be tailored to meet specific user requirements in a variety of applications.

An advantage of this arrangement is that vibration of the diaphragm is excited and energised by the flow of gas without requiring any additional energy source to provide atomising vibration.

It has been observed that positively biassing the diaphragm and lip into contact with one another provides improved performance compared with arrangements in which either the lip remains spaced from the diaphragm in the rest position or in which the lip merely rests in contact with the diaphragm.

Preferably the method includes the step of applying a tensioning force to the diaphragm such that it is maintained under tension in both the rest position and the displaced position.

It is particularly advantageous that the diaphragm should be held in tension in this way if the contact with the lip is minimal or non-existent.

Conveniently ambient air may be admitted to the outlet via an opening defined in the central portion of the diaphragm.

This provides the advantage that ambient air may be drawn through the nozzle to be mixed with the discharged gas, a particular advantage when delivering material for inhalation therapy. A further advantage is that it avoids the formation of a resonant cavity between the diaphragm and housing which would result in damping of the vibration.

The vibrational characteristics of the diaphragm may be modified by the addition of a mass connected to the diaphragm.

This enables the resonant frequency and damping of the diaphragm to be controlled.

The supply of gas may be provided by an air pump providing compressed air to an accumulating reservoir to be subsequently released by action of a trigger.

Alternatively a pressurised container containing pressurised gas may be connected via a metering valve to the chamber for the release of a pulse of gas in response to actuation of the trigger.

For applications such as humidifiers and fuel atomisation, a continuously operating supply of gas may be connected to the chamber.

According to a further aspect of the present invention there is disclosed apparatus for dispensing a flowable material comprising a nozzle, a gas supply for supplying pressurised gas to the nozzle such that a flow of gas is discharged in use through an aperture defined between a lip of the nozzle and a vibratable diaphragm, and dispensing means for introducing a quantity of the material into the flow whereby in use the material is entrained in the flow and atomised by the diaphragm, wherein the apparatus comprises a housing defining a chamber connected to the gas supply and communicating with an outlet defined by the nozzle via the aperture, the diaphragm constituting a wall of the chamber and being deformable in response to excess pressure in the chamber from a rest position to a displaced position in which the aperture is opened, and further comprising clamp means peripherally clamping the diaphragm such that the diaphragm is maintained under tension when deformed into the displaced position whereby the diaphragm is vibratable by the flow if gas, characterised in that the diaphragm is peripherally clamped by the clamp means such that an outer portion of the diaphragm defines said wall of the chamber, the lip being annular to thereby define a mouth of the outlet which is traversed by a central portion of the diaphragm.

Material in the form of a liquid may be dispensed by means of a displacement pump operable to dispense a metered volume of liquid.

Conveniently the dispensing means comprises a cylinder for receiving a quantity of liquid to be dispensed, a piston slidable in the cylinder to dispense liquid therefrom and an indexing mechanism operable to facilitate movement of the piston through predetermined incremental displacements at respective actuations of a trigger mechanism for the gas supply.

Preferred embodiments or the present invention will now be described by way of example only and with reference to the accompanying drawings of which
Figure 1 is a perspective part sectioned view of a nozzle in accordance with the present invention,
Figure 2 is a perspective view of a dispensing apparatus incorporating the nozzle of Figure 1,
Figure 3 is a schematic diagram showing the operation of the apparatus of Figure 2,
Figure 4 is a schematic diagram of an alternative apparatus,
Figure 5 is a perpective view of an alternative apparatus,
Figure 6 is a graphical representation of diaphragm displacment as a function of time in response to the release of a pulse of compressed gas through the aperture,
Figure 7 is a sectioned elevation of a diaphragm to which a quantity of particulate material has been added downstream of an annular aperture,
Figure 8 is a sectioned elevation of the nozzle and diaphragm schematically showing movement of the diaphragm to open the aperture,
Figure 9 is a sectioned elevation of an alternative arrangement in which the diaphragm is tensioned by a tensioning ring.
Figure 10 is a sectioned side elevation of a further alternative apparatus incorporating a circular diaphragm of the type shown generally with reference to Figure 1 and including a hand actuated compressor device for providing compressed air,
Figure 11 is a schematic front view of the apparatus of Figure 10, and
Figure 12 is a sectioned side elevation of a further alternative apparatus incorporating a pressurised dispensing container.

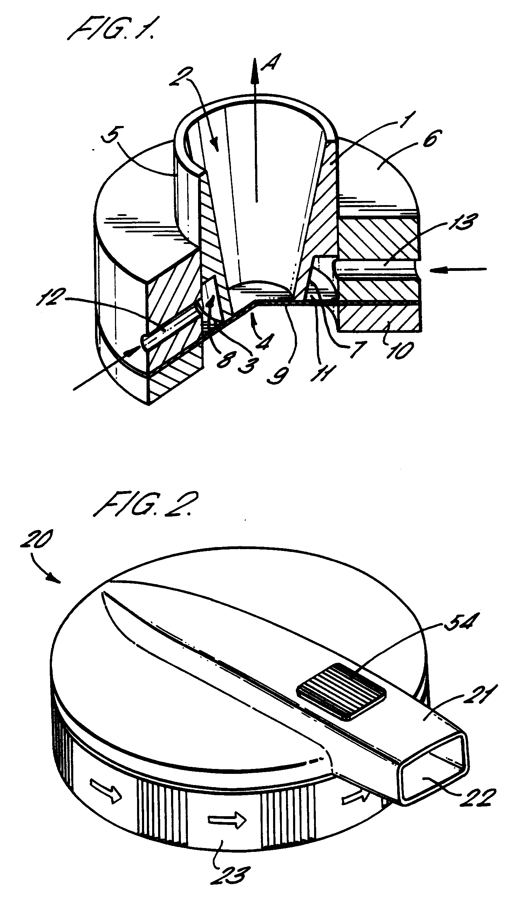

In Figure 1 a nozzle 1 defines an outlet 2 which is conically divergent in a direction of flow indicated by arrow A. The nozzle 1 terminates in an annular lip 3 defining a circular mouth 4 of the outlet 2. The nozzle 1 has a cylindrical outer surface 5 which is received in an annular housing 6, the surface 5 having a recessed portion 7 adjacent the lip 3 such that an annular chamber 8 is defined between the housing 6 and the recessed portion 7.

A diaphragm 9 is clamped between the housing 6 and an annular clamp portion 10 and in its relaxed state as shown in Figure 1 traverses the mouth 4 in contact with the lip 3. In this rest position an outer portion 11 of the diaphragm constitutes an annular wall of the chamber 8. In this closed position the chamber 8 is isolated from the outlet 2.

A gas inlet duct 12 extends radially through the housing 6 into communication with the chamber 8 and similarly a separate liquid inlet duct 13 communicates with the chamber 8 at a location circumferentially spaced from the gas inlet duct 12.

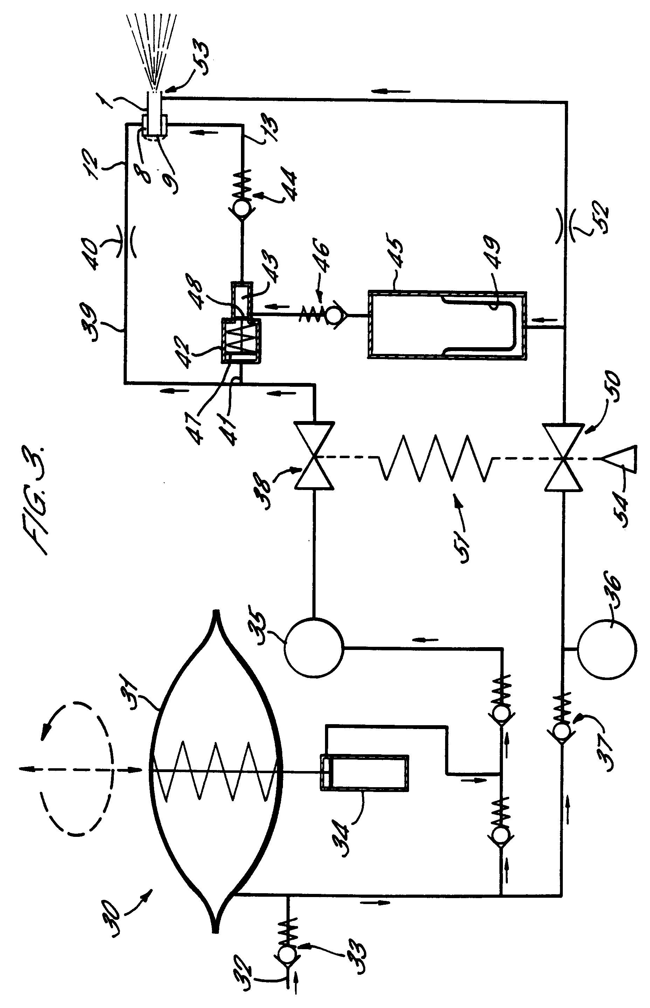

Figure 2 shows the external appearance of a dispensing apparatus 20 incorporating the nozzle 1 within a mouth piece 21 defining an airway 22 through which in use a user inhales. The apparatus 20 is also provided with an air inlet opening (not shown) allowing air to be drawn into the airway 22 in a manner such that gas emerging from the nozzle 1 will be entrained in the inhaled air flow during inhalation.

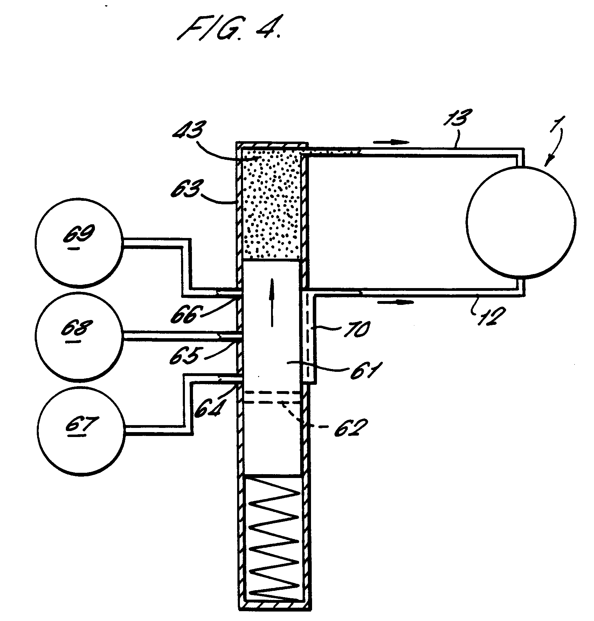

The internal working components of the apparatus 20 are illustrated schematically in Figure 3 which shows a diaphragm pump 30 of a type which is operated by cam action deriving axial reciprocation of a pump diaphragm 31 from manual rotation of a pump actuator 23 as indicated in Figure 2.

The pump 30 is connected to an air intake 32 via a one way valve 33 and delivers at each cycle of actuation a low pressure impulse of compressed air to a secondary compressor 34. The secondary compressor 34 is operated by movement of the actuator 23 180^{o} out of phase with the diaphragm pump 30 so as to provide a pressure boosted impulse of compressed air to a high pressure reservoir 35 which when charged contains air at about 5 bar.

The diaphragm pump 30 is also connected to a low pressure reservoir 36 via one way valve 37, air pressure of about 1.5 bar being held in the low pressure reservoir 36 when charged.

The diaphragm pump 30 includes a moulded face cam (not shown) which achieves during a single rotation of the actuator 23 a sequence of 4 axial cycles of the diaphragm to achieve the respective charged pressures referred to above.

The high pressure reservoir 35 is connected via a first valve 38 to a passageway 39 communicating with the chamber 8. The passageway 39 includes a flow restrictor 40 and communicates with a branch passageway 41 to which it is connected upstream of the flow restrictor. The branch passageway 41 communicates with a displacement pump 42 which is arranged to displace a metered volume of liquid from a metering chamber 43 in response to pressurised gas being received through the branch passageway.

The liquid inlet duct 13 communicates with the metering chamber 43 via a one way valve 44 and the metering chamber 43 is supplied with liquid from a liquid reservoir 45 via a further one way valve 46.

The displacement pump 42 has a first piston 47 which is moveable in response to pressurised gas received through the branch passageway 41 to move a second piston 48 arranged to expel liquid from the metering chamber 43. The first and second pistons 47 and 48 are spring loaded into a return position such that return travel of the second piston draws liquid into the metering chamber from the liquid reservoir 45.

The liquid reservoir 45 is partitioned by a flexible bag 49 containing the liquid to be dispensed.

A second valve 50 is operatively connected to the first valve 38 by a trigger mechanism 51. Compressed air is supplied via the second valve 50 from the low pressure reservoir 36 to the liquid reservoir 45 so as to externally pressurise the flexible bag 49. The liquid contents are thereby pressurised and this aids the transfer of liquid into the metering chamber 43.

The output of the second valve 50 is also connected by a further flow restrictor 52 to the outlet 2 of nozzle 1 via a low pressure air port 53 (not shown in Figure 1).

A trigger 54 is provided as part of the trigger mechanism 51 for operating the first and second valves 38 and 50 and is located on the apparatus 20 adjacent the mouth piece 21 as shown in Figure 2.

The trigger mechanism 51 is arranged to incorporate a spring mechanism operable such that actuation of the trigger 54 releases the second valve 50 and subsequently releases the first valve 38 thereby ensuring that the metering chamber 43 is filled with liquid and the pistons 47 and 48 are primed prior to release of the first valve 38.

In use, the user manually rotates the actuator 23 through 360^{o} thereby achieving pressurisation of both the high pressure reservoir 35 and the low pressure reservoir 36, this pressurisation being achieved as described above by a multi stage sequence in which the pump diaphragm 31 and secondary compressor 34 are repeatedly cycled.

The user presents the mouth piece 21 ready for inhalation through the airway 22 and manually depresses the trigger 54. The second valve 50 opens thereby externally pressurising the flexible bag 49 and charging the metering chamber 43 with liquid. The first valve 38 then opens and high pressure air delivered via the branch passageway 41 actuates the displacement pump 42 to deliver a metered dose of liquid into the chamber 8.

At the same time high pressured air is transmitted via the flow restrictor 40 to the gas inlet duct 12 and into the chamber 8.

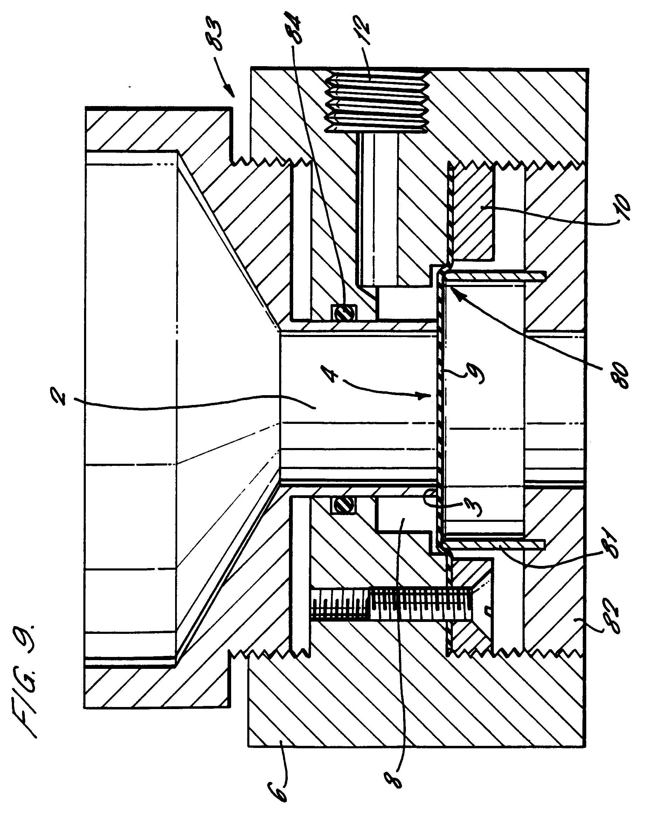

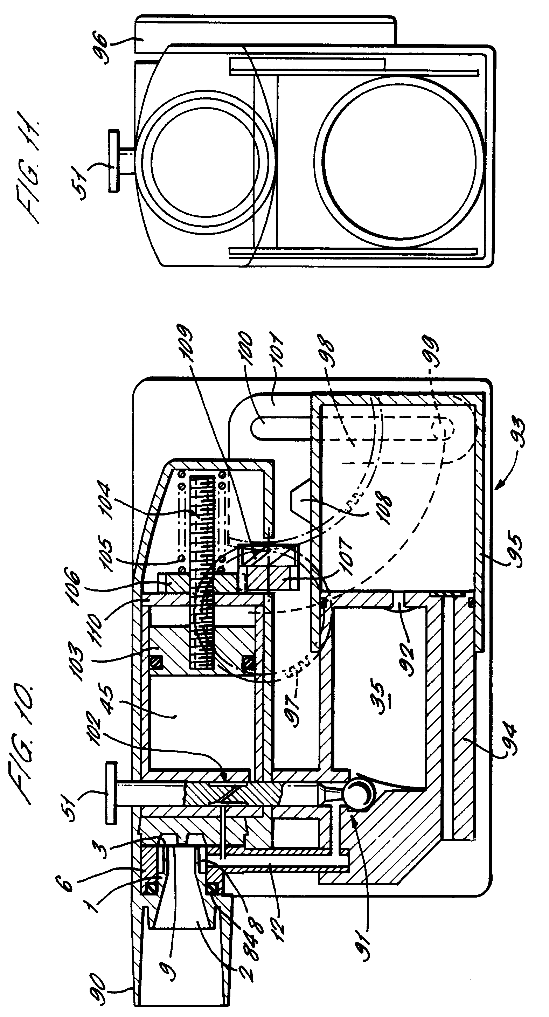

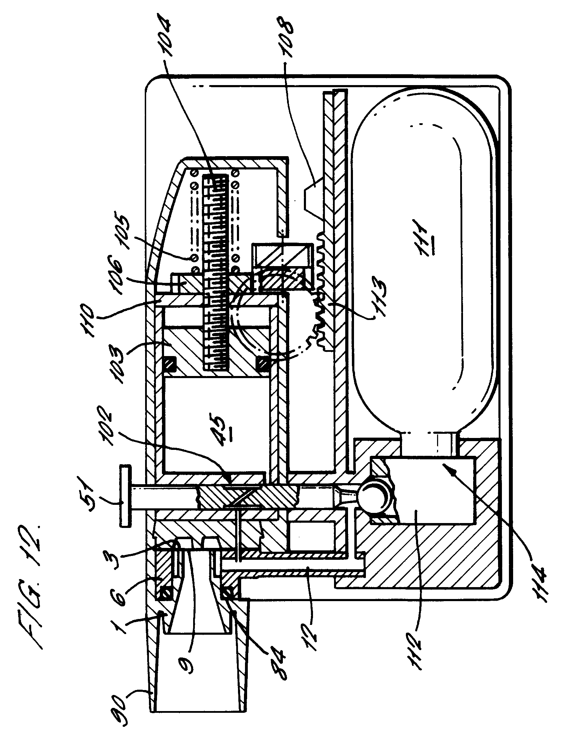

Excess pressure within the chamber 8 acting on the outer portion 11 of the diaphragm 9 unseats the diaphragm from the lip 3 of the nozzle 1 and both compressed air and liquid are ejected from the chamber 8 via an aperture 77 defined between the lip and the diaphragm into the outlet 2 as shown in Figure 8.

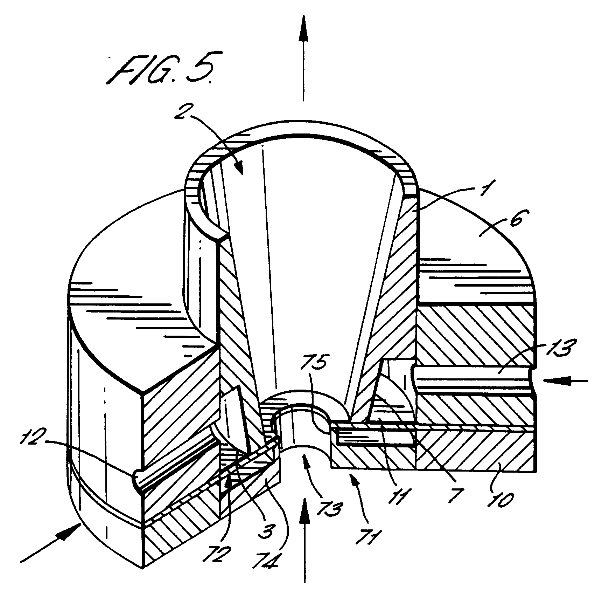

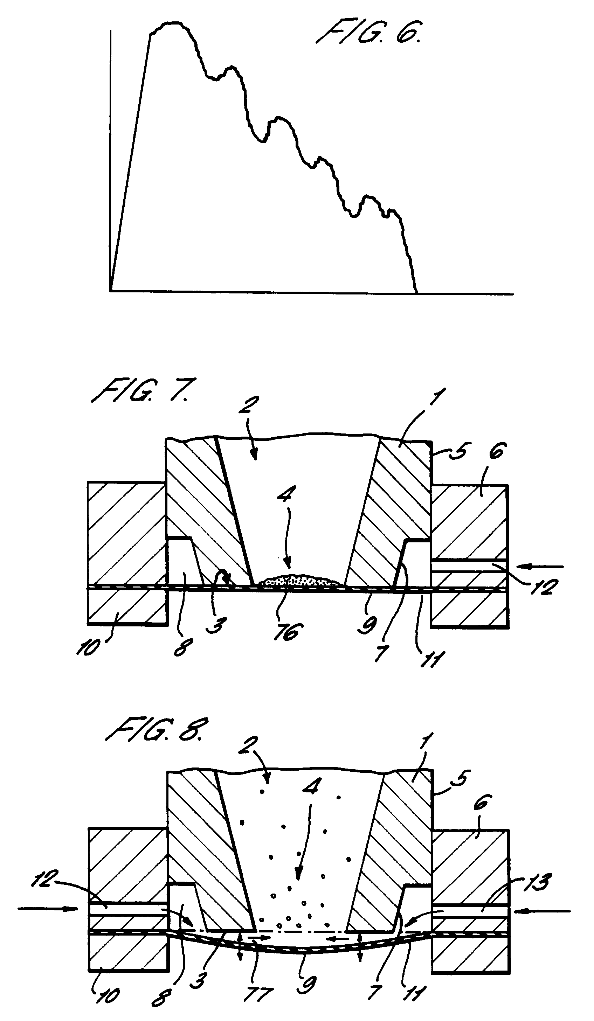

Figure 6 illustrates graphically the displacement of the diaphragm 9 away from the lip 3 as a function of time in response to a pulsed flow of gas being delivered to the chamber 8. The diaphragm 9 initially moves rapidly to its fully displaced position in response to an impulse of gas received within the chamber 8, the displacement of the diaphragm then progressively relaxing with decreasing gas pressure towards its rest position in which the aperture reduces to zero. A high frequency vibration of the diaphragm is superimposed on this displacement, the vibration being excited and energised by the flow of gas in a similar manner to that which is achieved in a conventional air horn such as those used in motor vehicles.

Liquid entrained in the flow of gas has been observed to exit the aperture between the lip 3 and the diaphragm 9 as a wall jet with an initial velocity which is directed radially inwardly, the jet then forming extended ligaments which break up into droplets.

The effect of the vibration present in the diaphragm 9 has been observed to be a marked reduction in the size of the droplets which are ultimately entrained in the air exiting from the outlet 2. The precise mechanism by which the vibration of the diaphragm 9 achieves this enhanced atomisation is believed to involve impact between the diaphragm and droplets and the effects of air currents created by the movement of the diaphragm.

A finely atomised mist of liquid is thereby produced in the airway 22 and is inhaled via mouth piece 21. The flow velocity of the inhaled air is boosted by a secondary flow of air received via low pressure air port 53. The oscillation of the diaphragm continues until the available air pressure is no longer sufficient to unseat the diaphragm, the time constant of the oscillation being much smaller than the time taken for the gas pressure to fully depleat.

In a prototype of the nozzle arrangement shown in Figure 1, a diaphragm having an exposed face of 8.5 mm diameter and cooperating with a lip of 6 mm diameter was found to operate satisfactorily when energised with an impulse of compressed air from a reservoir of 10 cc volume with an initial pressure of 5 bar. The diaphragm was found to oscillate at frequencies extending through the audible and into the ultrasonic range.

The duration of the air impulse was of the order of 1 second. It was found that a liquid could be atomised in such an arrangement such that 90% of droplets were of less than 20 microns diameter, 60% were less than 10 microns diameter and 30% below 5 microns.

Satisfactory redispersion of powders having single particle size of 3 microns was also achieved, the single particles being initially coalesced into larger granules and atomised during the dispensing operation. Dispensing such powders was achieved by orienting the nozzle 1 such that the outlet 2 extended upwardly from the diaphragm 9, particles of solid material being inserted into the outlet so as to initially rest on the diaphragm. The material in this situation was therefore introduced downstream of the aperture subsequently defined between the lip 3 and the diaphragm 9 in response to the gas flow. Dispersion of the particulate solid material in this arrangement is believed to be primarily as a result of impact between the diaphragm and the material, the material then being dispersed into a fine powder which becomes entrained in the gas flow. Droplets of liquid may similarly be dispersed and atomised by introducing the liquid so as to rest upon the diaphragm.

It should be noted that the illustration in Figure 8 of the opening of the aperture 77 is schematic and that in practice a complex mode of vibration set up in the diaphragm will result in the instantaneous cross section of the diaphragm having multiple peaks.

A modified arrangement is illustrated in Figure 4 which will now be described using corresponding reference numerals to those of preceding Figures where appropriate for corresponding features.

The modified apparatus 60 of Figure 4 has a nozzle 1 of the type shown in Figure 1 receiving compressed air via a gas inlet duct 12 and a supply of liquid via a liquid inlet duct 13.

A metering chamber 43 containing liquid receives a cylindrical piston 61 operable to displace the liquid and which includes a diametrically extending bore 62 allowing the piston 61 to also act as a shuttle valve for the release of compressed air. The piston 61 is slidable in a cylinder 63 having axially spaced air inlet ports 64, 65 and 66 connected to respective first, second and third high pressure air reservoirs 67, 68 and 69. Movement of the piston 61 along the cylinder 63 brings the bore 62 sequentially into registration with the first, second and third air inlet ports 64, 65 and 66 thereby constituting a valve means 61, 62, 63 allowing the compressed air to be conducted via an outlet port 70 into the gas inlet duct 12.

The valve means 61, 62, 63 may be incorporated into the apparatus of preceding Figures in order to deliver sequentially three separate impulses of compressed air during the discharge of liquid from the chamber 43. This modification takes account of the tendency for the liquid discharge to take longer to complete than the duration of an impulse of compressed air released from the high pressure reservoir.

In Figure 5 the nozzle 1 of Figure 1 is provided with an alternative diaphragm assembly 71 and will now be described using corresponding references to those of Figure 1 where appropriate for corresponding elements.

The arrangement of Figure 5 consists of a nozzle 1 defining an outlet 2 and an annular lip 3 defining a circular mouth 4 of the outlet.

The nozzle 1 is received in an annular housing 6 and an annular chamber 8 is defined between the housing and a recessed portion 7 of the outer surface 5 of the nozzle 1.

The diaphragm assembly 71 comprises a modified diaphragm 72 having a central opening 73 through which ambiant air may be drawn so as to flow through the outlet 2.

The diameter of the opening 73 is less than that of the internal diameter of the lip 3 so that the presence of the opening does not affect the operational relationship between the lip and the diaphragm 72.

A disc 74 is slidably received within the housing 6 immediately upstream of the diaphragm 72 and includes an axially projecting cylindrical projection 75 which is bonded to the diaphragm 72 peripherally of the opening 73.

The cylindrical projection 75 has an internal diameter equal to that of the opening 73 so that inhaled air may pass through the disc 74.

The disc 74 has the effect of adding a localised mass to the diaphragm 72 adjacent to its point of maximum vibrational movement and the effect of this additional mass is to modify the vibrational characteristics of the diaphragm and in particular has been found to be effective in reducing unwanted noise.

In an alternative arrangement (not shown) a disc may be added to the diaphragm without the presence of an opening 73 in order to modify its noise characteristics but without providing additional air flow through the diaphragm.

A further alternative is to provide the diaphragm 72 with an opening 73 but to not include disc 74 thereby providing additional air flow but without modifying the vibrational characteristics of the diaphragm.

In the above described arrangements the diaphragm 9 is peripherally clamped by a clamp portion 10 such that deformation of the diaphragm in response to the flow of gas to thereby define the aperture 77 results in the diaphragm being maintained under tension. The existence of this tension enables the vibration in the diaphragm to be sustained. It has been observed that the characteristics of vibration may be modified by varying the tension and to this end the diaphragm may be pre-loaded by means of a tensioning device 80 as illustrated in Figure 9 which will now be described using corresponding reference numerals to those of preceding Figures where appropriate for corresponding elements.

The tensioning device 80 comprises a tensioning ring 81 which is moved into contact with the diaphragm after the diaphragm has been clamped thereby deforming and tensioning the diaphragm in its rest position. The tensioning ring 81 is held by a screw adjustor 82 which is movable by a screw thread mechanism relative to the housing 6 to axially displace the tubular tensioning ring 81 relative to the clamp portion 10, thereby achieving a controlled amount of deformation of the diaphragm and thereby controlling tension.

Since this adjustment of the tensioning ring 81 axially displaces the location of the diaphragm within the housing 6, corresponding adjustment of the position of the lip 3 is desirable and in this example is facilitated by a screw threaded adjusting mechanism 83 whereby the nozzle 1 is axially adjustable relative to the housing 6 by rotating the nozzle to achieve axial adjustment.

An O-ring seal 84 is provided between the nozzle 1 and housing 6 to prevent the escape of gas from the chamber 8.

By adjusting the tension in the diaphragm 9 the vibrational characteristic and hence the atomising properties of the diaphragm 9 have been found to be adjustable so that the droplet size distribution present in the outlet 2 can be tailored to meet the requirements of particular products or uses of the device.

In the preferred embodiments described above the lip 3 is shown to contact the diaphragm 9 in the rest position of the diaphragm. This need not necessarily be the case since a small gap between the diaphragm and the lip in the rest position still provides a working arrangement, the diaphragm being vibratable provided that it is maintained under tension. The best results however have been obtained by maintaining the lip 3 in contact with the diaphragm in the rest position and adjusting the axial location at which the diaphragm is peripherally clamped such that a positive bias is exerted on the lip by the diaphragm, the bias being derived from tension established within the diaphragm.

In Figures 10 and 11 a further alternative apparatus is illustrated and is of a type which incorporates a circular diaphragm in a configuration which may be as shown in Figures 1, 5, 7, 8 or 9.

The apparatus of Figure 10 incorporates a nozzle 1 which is axially extended to form a mouth piece 90 suitable for oral inhalation. A circular diaphragm 9 is clamped in contact with a lip 3 of the nozzle 1 and is deformable to define an aperture communicating between a nozzle outlet 2 and an annular chamber 8 defined between the housing and the nozzle.

An inlet duct 12 communicates with the chamber 8 and is arranged to receive a flow of compressed air from a valve 91 actuated by depression of a trigger 51.

The valve 91 is arranged to release compressed air held within a reservoir 35 which receives air via a one way valve 92 from a displacement pump 93 which comprises a fixed piston 94 and a movable cylinder 95.

The pump 93 is actuated by manual rotation of a circular handle 96 as shown in Figure 12 which is connected to a first gear wheel 97 engaged to a geared quadrant 98 such that the quadrant 98 is rotated through 90^{o} by corresponding rotation of the handle 96.

Rotation of the quadrant 98 is translated to linear reciprocating motion of the cylinder 95 by means of a cam follower 99 mounted on the quadrant and received in a linear cam slot 100 formed in a plate 101 connected to the cylinder.

The trigger 51 is also arranged to release a metered dose of liquid from a liquid reservoir via a spool valve arrangement 102 allowing liquid to pass from the reservoir 45 into the inlet duct 12 when the trigger is depressed.

Liquid in the liquid reservoir 45 is pressurized by action of a piston 103 mounted on a lead screw 104 which is spring loaded by operation of a spring 105.

At each actuation of the trigger 51 the lead screw is allowed to move forward by an indexed amount determined by a priming operation effected by rotation of a driven gear 106 which is threadedly engaged on the lead screw 104 such that relative rotational movement between the drive gear 106 and the lead screw 104 is accompanied by relative axial displacement.

The driven gear 106 is peripherally engaged by an indexing gear 107 mounted on an axis parallel to the lead screw 104 and arranged such that rotation of the indexing gear through a predetermined angle results in rotation of the driven gear by an extent determining the required axial displacement of the lead screw to achieve the metered dose.

The indexing gear 107 is rotated by action of a detent 108 mounted on the cylinder 95 and engaging a coarse helical gear portion of the indexing gear such that at each axial reciprocation of the cylinder the indexing gear is caused to rotate through a predetermined angle.

In use the handle 96 is rotated so as to correspondingly turn the quadrant 98 through 90^{o}. This causes the cylinder to reciprocate relative to the fixed piston 94 thereby injecting compressed air into the reservoir 35 and at the same time resulting in rotation of the indexing gear 107 through a predetermined angle.

The drive gear 106 is correspondingly rotated and caused to move axially relative to the lead screw 104 in a direction away from an end stop 110 towards which it is biassed by action of the spring 105. During this priming operation the lead screw 104 is held stationary since the piston 104 cannot move while the spool valve 102 remains closed, the liquid within reservoir 45 being incompressable.

A user presents the mouth piece 90 to his mouth ready for inhalation and then depresses the trigger 51. The valve 91 is thereby opened to release compressed air from reservoir 35 through the inlet duct 12 to the chamber 8, the compressed air thereby acting on the diaphragm 9 to open an aperture between the diaphragm and the lip 3 and establish a discharge of air accompanied by vibration of the diaphragm.

At the same time the spool valve 102 releases liquid from the liquid reservoir 45 to be mixed with the flow of air within the inlet duct 12, liquid then being entrained in the discahrged air passing through the aperture. A wall jet of liquid under pressure emerges from the aperture between the lip 3 and the diaphragm 9 and travels generally parallel to the diaphragm, thereby being atomised by surface tension effects and by interaction with the vibrating diaphragm before being entrained in the flow of air passing out of the outlet into the mouth piece 90 for inhalation.

The discharge of liquid from the reservoir 45 is accompanied by an incremental movement of the piston 103, the travel of the piston being interrupted by engagement between the driven gear 106 and the end stop 110.

The trigger 51 is then returned to its initial position and the apparatus is again ready for use.

A further alternative apparatus is shown in Figure 13 which is based on the apparatus of Figures 11 and 12 but not including a piston and cylinder arrangement for compressing air. Instead, a pressurized container 111 charged with compressed gas is connected via a releasable connector 114 to a metering valve 112, the metering valve being operable by depression of the trigger 51 to discharge into the inlet duct 12 a metered pulse of compressed gas. The releasable connector 114 allows replacement containers to be fitted to replenish the supply of gas. Typically the compressed gas is carbon dioxide.

The apparatus is primed by rotation of handle 96 resulting in linear reciprocation of detent 108 via a linear rack 113 to index the driven gear 106 in readiness for dispensing a metered dose of liquid from liquid reservoir 45.

Operation of the apparatus of Figure 12 is otherwise similar to the operation of the apparatus of Figures 11 and 12 in that depression of the trigger 51 causes the release of both compressed gas and liquid into the inlet duct 12 to be dispensed via nozzle 1 into the mouth piece 90.

Further alternatives and modifications to the above apparatus and method are envisaged including an alternative arrangement in which liquid or powder is injected into the nozzle outlet by a dispensing means at a location downstream of the diaphragm.

Alternatively powder may be injected into the chamber 8 by a suitable mechanism, the powder being initially injected in either a single mass of coalesced particles or relatively coarse granules, the effect of being injected through the nozzle and subjected to the passage of the flow across the vibrating diaphragm being to micronise the powder.

The additional provision of a low pressure air flow to boost the rate of delivery of the atomised liquid or powder may be provided by injecting the secondary air flow into an annular space surrounding the nozzle. This secondary air flow may not be essential and it is envisaged that this step could be omitted from the above apparatus and method.

Apparatus intended for inhalation therapy may be provided with a breath actuated trigger in place of a manual actuator or may alternatively be provided with an automatically functioning trigger such as an electrical timer.

The apparatus may be provided as a disposable item or may include a re-usable portion into which a disposable reservoir of the material can be inserted.

The invention may be utilised in atomising material other than for inhalation therapy in which case an air duct other than a mouth piece would be provided.

Alternative mechanisms for metering the material may be employed such as conventional liquid metering pump arrangements.

The apparatus is conveniently moulded from plastics materials and the diaphragm may be a film comprising metal foil, polyester film, kapton film, or a laminate. Selection of the material will depend upon compatability with the material to be dispensed but in any event should be selected to be dimensionally stable and tear resistant.

The apparatus may be provided with an air control valve in order to adjust the speed of air discharged from the air reservoir to the nozzle.

The apparatus may be modified to provide for a purging impulse of air or gas to be delivered in a separate actuation following an actuation to deliver atomised material. The accumulation of residues in the apparatus may thereby be avoided. This is particularly useful when dispensing water based liquid products and especially those containing solids in suspension.

In the preferred embodiments described with reference to Figures 1 to 9 and Figures 10 to 12 refer to a single air inlet duct communicating with an annular chamber 8. It has in fact been found to be advantageous to provide a plurality of gas inlet ducts communicating with the chamber at circumferentially spaced locations in order to ensure that the gas pressure is applied evenly to the diaphragm. This has the advantage of avoiding the appearance of vortices in the flow within the outlet, the formation of such vortices being found to be detrimental to the atomisation process.

The apparatus described above with reference to Figures 1, 5, 8 and 9 may alternatively be utilised with a continuously operating supply of pressurised gas for use in such applications as fuel atomisation and humidifiers.

## Claims

1. A method of dispensing a flowable material comprising the steps of supplying pressurised gas to a nozzle (1) such that a flow of gas is discharged through an aperture (77) defined between a lip (3) of the nozzle and a diaphragm (9), introducing the material into the flow of gas such that material is entrained in the flow, and vibrating the diaphragm so as to atomise the material, the diaphragm constituting a wall of a chamber (8) communicating with the aperture, the diaphragm being deformable in response to excess gas pressure in the chamber from a rest position to a displaced position in which the aperture is opened to receive the flow passing between the lip and the diaphragm, the pressurised gas being supplied via the chamber such that the diaphragm is deformed into the displaced position and the diaphragm being peripherally clamped such that it is maintained under tension in the displaced position whereby the diaphragm is vibrated by the flow of gas, characterised in that the diaphragm has an outer portion which defines said wall of the chamber, the lip of the nozzle being annular thereby defining a mouth (4) of an outlet (2) defined by the nozzle and which is traversed by a central portion of the diaphragm in the rest position, and wherein the method includes the step of biasing the diaphragm into contact with the lip when the diaphragm is in its rest position.

2. A method as claimed in claim 1 including the step of applying a tensioning force to the diaphragm such that it is maintained under tension in both the rest position and the displaced position.

3. A method as claimed in claim 1 including the step of admitting ambient air to the outlet via an opening (73) defined in the central portion of the diaphragm.

4. A method as claimed in any of claims 1 to 3 including the step of modifying the vibrational characteristics of the diaphragm by a mass (74) connected to the diaphragm.

5. A method as claimed in any of claims 1 to 4 wherein the gas supply comprises an air pump (30), the method including the step of operating the air pump to accumulate a volume of compressed air and operating a trigger (54) to release the compressed air through a passageway (12) connected to the chamber.

6. A method as claimed in claim 5 including the step of containing the accumulated volume of compressed air in a plurality of reservoirs (67,68,69) and sequentially releasing the compressed air from the reservoirs to thereby extend the duration of the flow.

7. A method as claimed in any of claims 1 to 6 including the step of discharging a secondary flow of gas through the outlet such that the secondary flow is introduced into the flow of gas at a location downstream of the aperture with respect to the direction of flow.

8. A method as claimed in claim 7 including the step of deriving the secondary flow from a secondary gas supply at a lower pressure than that of the gas supplied to the chamber.

9. A method as claimed in any preceding claim wherein the material is a liquid and including the step of introducing the liquid into the flow at a location upstream of the aperture with respect to the direction of gas flow.

10. A method as claimed in any preceding claim wherein the material is a liquid and including the step of injecting a measured volume of the liquid into the flow by operation of a displacement pump (42).

11. A method as claimed in any of claims 1 to 8 including the step of introducing the material into the outlet at a location downstream of the closable aperture and in contact with the diaphragm.

12. A method as claimed in claim 11 wherein the material is a particulate solid in the form of loose or consolidated powder.

13. Apparatus for dispensing a flowable material comprising a nozzle (1), a gas supply (30) for supplying pressurised gas to the nozzle (1) such that a flow of gas is discharged in use through an aperture defined between a lip (3) of the nozzle and a vibratable diaphragm (9), and dispensing means (42) for introducing a quantity of the material into the flow whereby in use the material is entrained in the flow and atomised by the diaphragm, wherein the apparatus comprises a housing (6) defining a chamber (8) connected to the gas supply and communicating with an outlet (2) defined by the nozzle via the aperture, the diaphragm constituting a wall (11) of the chamber and being deformable in response to excess pressure in the chamber from a rest position to a displaced position in which the aperture is opened, and further comprising clamp means (10) peripherally clamping the diaphragm such that the diaphragm is maintained under tension when deformed into the displaced position whereby the diaphragm is vibratable by the flow of gas, characterised in that the diaphragm is peripherally clamped by the clamp means such that an outer portion of the diaphragm defines said wall of the chamber, the lip being annular to thereby define a mouth (4) of the outlet which is traversed by a central portion of the diaphragm.

14. Apparatus as claimed in claim 13 comprising tensioning means (18,82) operable to apply a tensioning force to the diaphragm in the rest position.

15. Apparatus as claimed in any of claims 13 and 14 wherein the diaphragm in its rest position is in contact with and exerts a biassing force on the lip.

16. Apparatus as claimed in claim 13 wherein the central portion of the diaphragm defines a central opening (73) communicating between ambient air and the outlet.

17. Apparatus as claimed in any of claims 13 to 16 comprising a mass (74) connected to the diaphragm.

18. Apparatus as claimed in any of claims 13 to 17 wherein the gas supply comprises an air pump (3) and reservoir means (35) operable to accumulate a volume of compressed air delivered by the pump, the apparatus further comprising a trigger (54) operable to release the compressed air to flow to the chamber via a passageway (39) defined in the housing.

19. Apparatus as claimed in claim 18 wherein the reservoir means comprises a plurality of reservoirs (67,68,69) and further comprising valve means (61,62,63) operable to release the compressed air sequentially therefrom to thereby extend the duration of the flow.

20. Apparatus as claimed in any of claims 13 to 19 wherein the gas supply comprises connector means (114) for releasably connecting a pressurised gas container (111) in communication with the chamber via a valve operable to release a pulse of compressed gas.

21. Apparatus as claimed in any of claims 13 to 20 wherein the gas supply comprises supply means for continuously supplying pressurised gas to the chamber.

22. Apparatus as claimed in any of claims 13 to 21 comprising an inlet duct (13) for introducing the material into the flow and communicating with the chamber at a location upstream of the closable aperture with respect to the direction of gas flow.

23. Apparatus as claimed in claim 22 for use in dispensing material in the form of liquid wherein the dispensing means comprises a displacement pump (42) operable to dispense a metered volume of liquid.

24. Apparatus as claimed in claim 23 wherein the dispensing means comprises a cylinder for receiving a quantity of liquid to be dispensed, a piston (103) slidable in the cylinder to displace liquid therefrom and an indexing mechanism (104,105,106,107,108) operable to facilitate movement of the piston through predetermined incremental displacements at respective actuations of a trigger mechanism for the gas supply.

25. Apparatus as claimed in any of claims 13 to 23 including secondary discharge means (36,53) for discharging a secondary flow of gas through the outlet at a location downstream of the aperture with respect to the direction of gas flow.

26. Apparatus as claimed in claim 25 wherein the secondary discharge means comprises a secondary gas supply (36) operable to supply gas at a lower pressure than that supplied to the chamber.

## Patentansprüche

1. Verfahren zum Ausgeben von fließfähigem Material, umfassend die Schritte: Zuführen von Druckgas zu einer Düse (1) derart, daß ein Gasstrom durch eine zwischen einer Lippe (3) der Düse und einer Membrane (9) festgelegte Öffnung (77) abgegeben wird, Einführen des Materials in den Gasstrom derart, daß Material in dem Strom mitgenommen wird, und in Schwingung versetzen der Membrane zum Zerstäuben des Materials, wobei die Membrane eine Wand einer mit der Öffnung kommunizierenden Kammer (8) bildet, wobei die Membrane in Antwort auf Überschußgasdruck in der Kammer von einer Ruhestellung zu einer verlagerten Stellung verformbar ist, in der die Öffnung zur Aufnahme des zwischen der Lippe und der Membrane fließenden Stroms geöffnet ist, wobei das Druckgas über die Kammer derart zugeführt wird, daß die Membrane in die verlagerte Stellung verformt wird, und wobei die Membrane am Umfang derart festgeklemmt ist, daß sie in der verlagerten Stellung unter Spannung gehalten wird, wodurch die Membrane durch den Gasstrom in Schwingung versetzt wird, **dadurch gekennzeichnet**, daß die Membrane einen Außenabschnitt aufweist, der die Wand der Kammer festlegt, wobei die Lippe der Düse ringförmig ist, um hierdurch einen Mund (4) eines Auslasses (2) festzulegen, der durch die Düse festgelegt ist und der in der Ruhestellung von einem Mittelabschnitt der Membrane überspannt wird, und wobei das Verfahren den Schritt beinhaltet, die Membrane in Kontakt mit der Lippe zu spannen, wenn sich die Membrane in ihrer Ruhestellung befindet.

2. Verfahren nach Anspruch 1, welches den Schritt beinhaltet, auf die Membrane eine derartige Zugkraft auszuüben, daß sie sowohl in der Ruhestellung als auch in der verlagerten Stellung unter Spannung gehalten wird.

3. Verfahren nach Anspruch 1, welches den Schritt beinhaltet, über eine in dem Mittelabschnitt der Membrane festgelegte Öffnung (73) Umgebungsluft zu dem Auslaß gelangen zu lassen.

4. Verfahren nach einem der Ansprüche 1 bis 3, welches den Schritt beinhaltet, die Schwingeigenschaften der Membrane durch eine mit der Membrane verbundene Masse (74) zu modifizieren.

5. Verfahren nach einem der Ansprüche 1 bis 4, wobei die Gaszufuhr eine Luftpumpe (30) umfaßt, wobei das Verfahren den Schritt beinhaltet, die Luftpumpe zum Ansammeln eines Druckluftvolumens zu betätigen und einen Auslöser (54) zu betätigen, um die Druckluft durch eine mit der Kammer verbundene Passage (12) abzulassen.

6. Verfahren nach Anspruch 5, welches den Schritt beinhaltet, das angesammelte Druckluftvolumen in einer Mehrzahl von Reservoirs (67, 68, 69) aufzunehmen und die Druckluft aus den Reservoirs nacheinander abzulassen, um hierdurch die Fließdauer zu verlängern.

7. Verfahren nach einem der Ansprüche 1 bis 6, welches den Schritt beinhaltet, einen sekundären Gasstrom durch den Auslaß derart abzugeben, daß der sekundäre Strom in den Gasstrom an einer Stelle stromab der Öffnung relativ zur Strömungsrichtung eingeführt wird.

8. Verfahren nach Anspruch 7, welches den Schritt beinhaltet, den sekundär ren Strom aus einer sekundären Gaszufuhr mit einem geringeren Druck als jenem des der Kammer zugeführten Gases abzuleiten.

9. Verfahren nach einem der vorhergehenden Ansprüche, wobei das Material eine Flüssigkeit ist und welches den Schritt beinhaltet, die Flüssigkeit in den Strom an einer Stelle stromauf der Öffnung relativ zur Richtung des Gasstroms einzuführen.

10. Verfahren nach einem der vorhergehenden Ansprüche, wobei das Material eine Flüssigkeit ist, und welches den Schritt beinhaltet, zur Betätigung einer Verdrängungspumpe (42) ein dosiertes Flüssigkeitsvolumen in den Strom einzuspritzen.

11. Verfahren nach einem der Ansprüche 1 bis 8, welches den Schritt beinhaltet, das Material in den Auslaß an einer Stelle stromab der verschließbaren Öffnung und in Kontakt mit der Membrane einzuführen.

12. Verfahren nach Anspruch 11, wobei das Material partikelförmiger Feststoff in Form von losem oder verfestigtem Pulver ist.

13. Vorrichtung zum Ausgeben von fließfähigem Material, umfassend: eine Düse (1), eine Gaszufuhr (30) zum Zuführen von Druckgas zu der Düse (1) derart, daß bei Verwendung ein Gasstrom durch eine zwischen einer Lippe (3) der Düse und einer in Schwingung versetzbaren Membrane (9) festgelegte Öffnung abgegeben wird, sowie ein Ausgabemittel (42) zum Einführen einer Menge des Materials in den Strom, wodurch bei Verwendung das Material in dem Strom mitgenommen und durch die Membrane zerstäubt wird, wobei die Vorrichtung ein Gehäuse (6) aufweist, welches eine Kammer (8) festlegt, die mit der Gaszufuhr verbunden ist und über die Öffnung mit einem durch die Düse festgelegten Auslaß (2) kommuniziert, wobei die Membrane eine Wand (11) der Kammer bildet und in Antwort auf Überschußdruck in der Kammer von einer Ruhestellung zu einer verlagerten Stellung verformbar ist, in der die Öffnung geöffnet ist, und ferner umfassend ein Klemmittel (10), das die Membrane am Umfang derart festklemmt, daß die Membrane bei Verformung in die verlagerte Stellung unter Spannung gehalten wird, wodurch die Membrane durch den Gasstrom in Schwingung versetzbar ist, **dadurch gekennzeichnet**, daß die Membrane am Umfang durch das Klemmittel derart festgeklemmt ist, daß ein Außenabschnitt der Membrane die Wand der Kammer festlegt, wobei die Lippe ringförmig ist, um einen Mund (4) des Auslasses festzulegen, der von einem Mittelabschnitt der Membrane überspannt ist.

14. Vorrichtung nach Anspruch 13, umfassend ein Spannmittel (18, 82), das betätigbar ist, um in der Ruhestellung auf die Membrane eine Zugkraft auszuüben.

15. Vorrichtung nach einem der Ansprüche 13 und 14, wobei die Membrane in ihrer Ruhestellung mit der Lippe in Kontakt steht und auf diese eine Spannkraft ausübt.

16. Vorrichtung nach Anspruch 13, wobei der Mittelabschnitt der Membrane eine Mittelöffnung (73) festlegt, die eine Verbindung zwischen der Umgebungsluft und dem Auslaß herstellt.

17. Vorrichtung nach einem der Ansprüche 13 bis 16, die eine mit der Membrane verbundene Masse (74) aufweist.

18. Vorrichtung nach einem der Ansprüche 13 bis 17, wobei die Gaszufuhr eine Luftpumpe (3) sowie ein Reservoirmittel (35) aufweist, das betätigbar ist, um ein von der Pumpe geliefertes Druckluftvolumen anzusammeln, wobei die Vorrichtung ferner einen Auslöser (54) aufweist, der betätigbar ist, um die Druckluft abzulassen, so daß sie über eine in dem Gehäuse festgelegte Passage (39) zu der Kammer fließt.

19. Vorrichtung nach Anspruch 18, wobei das Reservoirmittel eine Mehrzahl von Reservoirs (67, 68, 69) umfaßt und die ferner ein Ventilmittel (61, 62, 63) umfaßt, das betätigbar ist, um die Druckluft aus diesen nacheinander abzulassen, um hierdurch die Fließdauer zu verlängern.

20. Vorrichtung nach einem der Ansprüche 13 bis 19, wobei die Gaszufuhr ein Verbindungsmittel (114) aufweist, um lösbar einen Druckgasbehälter (111) über ein Ventil, welches betätigbar ist, um ein Druckgasimpuls abzulassen, mit der Kammer in Verbindung zu bringen.

21. Vorrichtung nach einem der Ansprüche 13 bis 20, wobei die Gaszufuhr ein Zufuhrmittel umfaßt, um der Kammer fortlaufend Druckgas zuzuführen.

22. Vorrichtung nach einem der Ansprüche 13 bis 21, umfassend eine Einlaßleitung (13) zum Einführen des Materials in den Strom und zur Verbindung mit der Kammer an einer Stelle stromauf der verschließbaren Öffnung relativ zur Richtung des Gasstroms.

23. Vorrichtung nach Anspruch 22 zur Verwendung bei der Ausgabe von in flüssiger Form vorliegendem Material, wobei das Ausgabemittel eine Verdrängerpumpe (42) umfaßt, die betätigbar ist, um ein dosiertes Flüssigkeitsvolumen auszugeben.

24. Vorrichtung nach Anspruch 23, wobei das Ausgabemittel einen Zylinder zur Aufnahme einer auszugebenden Flüssigkeitsmenge umfaßt, einen Kolben (103), der in dem Zylinder zum Verdrängen Von Flüssigkeit aus diesem verschiebbar ist, sowie einen Indexiermechanismus (104, 105, 106, 107, 108), der betätigbar ist, um die Bewegung des Kolbens über vorbestimmte stufenweise Hübe bei jeweiligen Betätigungen eines Auslösermechanismus für die Gaszufuhr zu erleichtern.

25. Vorrichtung nach einem der Ansprüche 13 bis 23, umfassend ein sekundäres Ausgabemittel (36, 53) zum Ausgeben eines sekundären Gasstroms durch den Auslaß an einer Stelle stromab der Öffnung relativ zur Richtung des Gasstroms.

26. Vorrichtung nach Anspruch 25, wobei das sekundäre Ausgabemittel eine sekundäre Gaszufuhr (36) umfaßt, die betätigbar ist, um Gas bei einem geringeren Druck als dem der Kammer zugeführten Druck zuzuführen.

## Revendications

1. Procédé de distribution d'une matière pouvant couler, comprenant les étapes dans lesquelles on alimente en gaz comprimé une buse (1) de manière qu'un écoulement de gaz soit déchargé à travers une ouverture (77) définie entre une lèvre (3) de la buse et une membrane (9), on introduit la matière dans l'écoulement de gaz afin que la matière soit entraînée dans l'écoulement, et on fait vibrer la membrane de manière à atomiser la matière, la membrane constituant une paroi d'une chambre (8) communiquant avec l'ouverture, la membrane pouvant être déformée en réponse à une pression gazeuse excessive dans la chambre depuis une position de repos jusqu'à une position déplacée dans laquelle l'ouverture est dégagée pour recevoir l'écoulement passant entre la lèvre et la membrane, le gaz comprimé étant amené en passant dans la chambre afin que la membrane soit amenée par déformation dans la position déplacée, et la membrane étant serrée à sa périphérie afin d'être maintenue tendue dans la position déplacée, grâce à quoi l'écoulement de gaz fait vibrer la membrane,
caractérisé en ce que la membrane comporte une partie extérieure qui définit ladite paroi de la chambre, la lèvre de la buse étant annulaire, définissant ainsi une embouchure (4) d'une sortie (2) définie par la buse et qui est traversée par une partie centrale de la membrane dans la position de repos, le procédé comprenant l'étape de rappel de la membrane en contact avec la lèvre lorsque la membrane est dans sa position de repos.

2. Procédé selon la revendication 1, comprenant l'étape d'application d'une force de tension à la membrane afin qu'elle soit maintenue tendue à la fais dans la position de repos et dans la position déplacée.

3. Procédé selon la revendication 1, comprenant l'étape d'admission d'air ambiant à la sortie en passant par un trou (73) défini dans la partie centrale de la membrane.

4. Procédé selon l'une quelconque des revendications 1 à 3, comprenant l'étape de modification des caractéristiques de vibration de la membrane au moyen d'une masse (74) reliée à la membrane.

5. Procédé selon l'une quelconque des revendications 1 à 4, dans lequel l'alimentation en gaz comprend une pompe à air (30), le procédé comprenant l'étape d'actionnement de la pompe à air pour accumuler un volume d'air comprimé et d'actionnement d'une détente (54) pour libérer l'air comprimé dans un passage (12) raccordé à la chambre.

6. Procédé selon la revendication 5, comprenant l'étape consistant à contenir le volume accumulé d'air comprimé dans plusieurs réservoirs (67, 68, 69) et à libérer séquentiellement l'air comprimé depuis les réservoirs pour augmenter ainsi la durée de l'écoulement.

7. Procédé selon l'une quelconque des revendications 1 à 6, comprenant l'étape de décharge d'un écoulement secondaire de gaz à travers la sortie afin que l'écoulement secondaire soit introduit dans l'écoulement de gaz en un emplacement en aval de l'ouverture par rapport au sens d'écoulement.

8. Procédé selon la revendication 7, comprenant l'étape de dérivation d'écoulement secondaire à partir d'une alimentation en gaz secondaire à une pression inférieure à celle du gaz fourni à la chambre.

9. Procédé selon l'une quelconque des revendications précédentes, dans lequel la matière est un liquide, et comprenant l'étape d'introduction du liquide dans l'écoulement en un emplacement en amont de l'ouverture par rapport au sens d'écoulement du gaz.

10. Procédé selon l'une quelconque des revendications précédentes, dans lequel la matière est un liquide, et comprenant l'étape d'injection d'un volume mesuré du liquide dans l'écoulement par l'actionnement d'une pompe volumétrique (42).

11. Procédé selon l'une quelconque des revendications 1 à 8, comprenant l'étape d'introduction de la matière dans la sortie en un emplacement en aval de l'ouverture pouvant être fermée, et en contact avec la membrane.

12. Procédé selon la revendication 11, dans lequel la matière est une matière solide en particules sous la forme d'une poudre inconsistante ou tassée.

13. Appareil pour distribuer une matière pouvant couler comportant une buse (1), une alimentation en gaz (30) pour alimenter en gaz comprimé la buse (1) afin qu'un écoulement de gaz soit déchargé, lors de l'utilisation, à travers une ouverture définie entre une lèvre (3) de la buse et une membrane vibrante (9), et des moyens de distribution (42) destinés à introduire une quantité de la matière dans l'écoulement grâce à quoi, lors de l'utilisation, la matière est entraînée dans l'écoulement et atomisée par la membrane, l'appareil comportant un corps (6) définissant une chambre (8) raccordée à l'alimentation en gaz et communiquant avec une sortie (2) définie par la buse, par l'intermédiaire de l'ouverture, la membrane constituant une paroi (11) de la chambre et pouvant être déformée en réponse à une pression excessive dans la chambre depuis une position de repos jusqu'à une position déplacée dans laquelle l'ouverture est dégagée, et comportant en outre des moyens de serrage (10) serrant à sa périphérie la membrane afin que la membrane soit maintenue tendue lorsqu'elle est déformée dans la position déplacée, grâce à quoi l'écoulement de gaz fait vibrer la membrane, caractérisé en ce que la membrane est serrée à sa périphérie par les moyens de serrage de manière qu'une partie extérieure de la membrane définisse ladite paroi de la chambre, la lèvre étant annulaire pour définir ainsi une embouchure (4) de la sortie qui est traversée par une partie centrale de la membrane.

14. Appareil selon la revendication 13, comportant des moyens tendeurs (18, 82) pouvant être mis en oeuvre pour appliquer une force de tension à la membrane dans la position de repos.

15. Appareil selon l'une des revendications 13 et 14, dans lequel la membrane dans sa position de repos est en contact avec la lèvre et exerce une force de rappel sur celle-ci.

16. Appareil selon la revendication 13, dans lequel la partie centrale de la membrane définit un trou central (73) établissant une communication entre l'air ambiant et la sortie.

17. Appareil selon l'une quelconque des revendications 13 à 16, comportant une masse (74) reliée à la membrane.

18. Appareil selon l'une quelconque des revendications 13 à 17, dans lequel l'alimentation en gaz comprend une pompe à air (3) et un moyen à réservoir (35) pouvant être mis en oeuvre pour accumuler un volume d'air comprimé distribué par la pompe, l'appareil comportant en outre une détente (54) pouvant être actionné pour libérer l'air comprimé afin qu'il s'écoule vers la chambre en empruntant un passage (39) défini dans le corps.

19. Appareil selon la revendication 18, dans lequel le moyen à réservoir comporte plusieurs réservoirs (67, 68, 69), et comportant en outre un moyen à valve (61, 62, 63) pouvant être actionné pour en libérer séquentiellement l'air comprimé de façon à prolonger ainsi la durée de l'écoulement.

20. Appareil selon l'une quelconque des revendications 13 à 19, dans lequel l'alimentation en gaz comprend un moyen de raccordement (114) destiné à raccorder de façon libérable un conteneur (111) à gaz comprimé en communication avec la chambre en passant par un clapet pouvant être actionné pour libérer une pulsation de gaz comprimé.

21. Appareil selon l'une quelconque des revenu dications 13 à 20, dans lequel l'alimentation en gaz comprend un moyen d'alimentation pour alimenter en continu la chambre en gaz comprimé.

22. Appareil selon l'une quelconque des revendications 13 à 21, comportant un canal d'entrée (13) pour l'introduction de la matière dans l'écoulement et communiquant avec la chambre en un emplacement en amont de l'ouverture pouvant être fermée, par rapport au sens d'écoulement du gaz.

23. Appareil selon la revendication 22 à utiliser dans la distribution d'une matière sous la forme d'un liquide, dans lequel le moyen de distribution comprend une pompe volumétrique (32) pouvant être actionnée pour distribuer un volume dosé de liquide.

24. Appareil selon la revendication 23, dans lequel le moyen de distribution comprend un cylindre destiné à recevoir une quantité de liquide devant être distribuée, un piston (103) pouvant coulisser dans le cylindre pour en chasser le liquide et un mécanisme d'indexage (104, 105, 106, 107, 108) pouvant être mis en oeuvre pour faciliter un mouvement du piston sur des déplacements à accroissements prédéterminés lors d'actionnements respectifs d'un mécanisme de détente pour l'alimentation en gaz.

25. Appareil selon l'une quelconque des revendications 13 à 23, comprenant un moyen de décharge secondaire (36, 53) destiné à décharger un écoulement du gaz secondaire à travers la sortie en un emplacement en aval de l'ouverture par rapport au sens d'écoulement de gaz.

26. Appareil selon la revendication 25, dans lequel le moyen de décharge secondaire comporte une alimentation (36) en gaz secondaire pouvant être mise en oeuvre pour une alimentation en gaz à une pression plus basse que celle fournie à la chambre.
